# EUROPEAN PATENT APPLICATION

(11) **EP 4 666 957 A1**
(43) Date of publication of application: **24.12.2025**
(21) Application number: 25183708.4
(22) Date of filing: 18.06.2025
(51) Int. Cl.: A61B 8/06, A61B 8/00, A61B 8/08

(54) **A BLOOD FLOW MONITORING DEVICE**

(30) Priority: 19.06.2024 CN 202410795807
(71) Applicant: ChinaBridge (Shenzen) Medical Technology Co., Ltd., Shenzhen, Guangdong 518102 (CN)
(72) Inventor: WANG, Weiyun, Shenzhen Guangdong, 518102 (CN)
(74) Representative: Pfenning, Meinig & Partner mbB

(57) **Abstract**

A blood flow monitoring device includes a monitoring equipment and a supporting mechanism. The monitoring equipment includes a first housing with a first shell, a monitoring unit with a second shell connected to the first shell, a flow velocity measurement probe arranged in the second shell, a scanning positioning probe arranged in the second shell, a driving mechanism in the first housing, and a control mechanism in the first housing. The supporting mechanism configured to be arranged on a surface of a living body limb. The first housing is movably sleeved on the supporting mechanism, and the first shell reciprocates on the supporting mechanism. The flow velocity measurement probe is configured to calculate the blood flow rate using the blood flow velocity and a blood vessel diameter. The scanning positioning probe is configured to monitor the position and diameter of the blood vessel using the second corresponding ultrasonic signal.

## Description

### CROSS REFERENCE TO RELATED APPLICATIONS

The present application claims priority to Chinese Patent Application No. 202410795807.2, filed June 19, 2024, the entire disclosure of which is incorporated herein by reference.

### TECHNICAL FIELD

The embodiments of the present application belong to the technical field of blood flow monitoring, and in particular, relate to a blood flow monitoring device.

### BACKGROUND ART

Extracorporeal membrane oxygenation (ECMO) is mainly used to provide continuous extracorporeal respiration and circulation to patients with severe cardiopulmonary failure to maintain their lives. The core parts of ECMO are the membrane lung (artificial lung) and the blood pump (artificial heart). When the ECMO device is in operation, blood is drawn from the human body, oxygen is exchanged and absorbed through the membrane lung, and carbon dioxide is discharged. The blood that has undergone gas exchange is returned to the human body under the promotion of the blood pump.

There are two modes of ECMO establishment: VA and VV cannulation. When VA femoral arteriovenous cannulation is used, the femoral artery cannulation affects the circulation of the artery at the cannulation site, which can easily lead to insufficient blood supply to the distal artery of the lower limb, and symptoms of ischemia will occur on the side of the arterial cannulation. In severe cases, compartment syndrome and limb gangrene and other malignant events may occur. In clinical practice, a distal perfusion catheter is connected to the side wall of the cannula with a connector, and the catheter is inserted into the distal blood vessel at the incision for distal perfusion. Insufficient blood supply can cause symptoms of ischemia, pale skin color, decreased temperature, damage to the limb, and even the need for amputation; excessive blood supply can cause swelling of the distal limb, requiring incision treatment, causing more harm to the patient. For this purpose, a device that can continuously monitor limb blood flow for a long time is needed.

Existing technology relies on ultrasonic Doppler equipment (blood flow detector) to monitor the flow in the patient's distal blood vessels once, which brings a greater workload to medical staff and cannot perform continuous monitoring. If there is a problem of insufficient blood supply or excessive blood supply that cannot be discovered in time, it can result in the inability to provide timely treatment and cause serious consequences.

The existing technology usually uses ultrasonic image processing to measure the blood vessel diameter, such as patent CN114820587A, but ultrasonic image processing requires complex algorithms and a high-performance computing unit to achieve it. In addition, such algorithm equipment and ultrasonic probes use multiple sets of crystal oscillators, which are bulky and not suitable for being fixed on the surface of a limb for continuous monitoring. Therefore, a lightweight, compact device that can simply calculate the position and the diameter blood vessels is needed to solve this problem.

### SUMMARY OF THE INVENTION

In order to solve or alleviate the problems in the prior art, the present invention relates to a blood flow monitoring device, which is mainly used in the field of human blood flow monitoring, and more specifically in the field of continuous blood flow monitoring of limbs.

A blood flow monitoring device provided in an embodiment of the present application specifically includes a monitoring device and a supporting mechanism;
The support mechanism is arranged on the surface of the living body limb;
The monitoring device comprises a first housing and a monitoring unit, a driving mechanism and a control unit arranged in the first housing, the control unit is connected to the driving mechanism and the monitoring unit respectively, and the first housing is movably sleeved on the supporting mechanism;
The monitoring unit comprises a second shell, a flow velocity measurement probe and a scanning positioning probe, wherein the flow velocity measurement probe and the scanning positioning probe are both arranged in the second shell, and the second shell is connected to the first shell;
The flow velocity measurement probe is used to transmit a first ultrasonic signal at a preset inclination angle to the direction of the blood flow velocity and receive a first corresponding ultrasonic signal reflected after monitoring the blood, measure the blood flow velocity in the blood vessel through the first corresponding ultrasonic signal, and calculate the blood flow rate through the blood flow velocity and the blood vessel diameter;
The scanning positioning probe is used to transmit a second ultrasonic signal perpendicular to the blood flow direction and the first ultrasonic signal and receive a second corresponding ultrasonic signal reflected after monitoring the blood, and monitor the position and diameter of the blood vessel through the second ultrasonic signal;
The driving mechanism is connected to the first shell and is controlled by the control mechanism to drive the first shell to reciprocate on the support mechanism to determine the position of the target blood vessel and to monitor the blood flow in the target blood vessel.

As a preferred embodiment of the present application, the driving unit includes a power output mechanism and a first transmission mechanism connected to each other, a second transmission mechanism is provided on the supporting mechanism, the monitoring unit and the power output mechanism are electrically connected to the control mechanism respectively, the first transmission mechanism is connected to the first housing, and the first transmission mechanism and the second transmission mechanism are meshed;
The control unit controls the power output mechanism to drive the first transmission mechanism to move, and the first transmission mechanism cooperates with the second transmission mechanism to drive the first housing to reciprocate on the support mechanism.

As a preferred embodiment of the present application, the support mechanism comprises a first support member, a second support member, a third support member and a fourth support member which are sequentially connected to each other, and the first support member is connected to the fourth support member;
The first support member and the third support member are both arc-shaped and are arranged in a direction perpendicular to the blood flow direction. The first support member and the third support member are both provided with a second transmission mechanism;
The second supporting member and the fourth supporting member are both arranged in a direction parallel to the blood flow direction.

As a preferred embodiment of the present application, the first transmission mechanism includes a first gear, a second gear, a third gear and a transmission shaft;
The first gear is connected to the output end of the power output mechanism, the second gear and the third gear are respectively arranged on the transmission shaft and are both coaxially arranged with the transmission shaft, and the first gear and the second gear are meshed;
Both ends of the transmission shaft are rotatably arranged on the inner side wall of the first shell, and the third gear cooperates with the second transmission mechanism to drive the monitoring device to reciprocate on the supporting mechanism.

As a preferred embodiment of the present application, two groups of first grooves are provided on the second gear, and extreme position detection mechanisms are provided at corresponding positions of the two groups of first grooves, and the extreme position detection mechanisms are used to detect whether the monitoring device moves to the two extreme positions of the support mechanism.

As a preferred embodiment of the present application, the flow velocity measurement probe includes a first transmitting ultrasonic crystal oscillator and a first receiving ultrasonic crystal oscillator, the first transmitting ultrasonic crystal oscillator and the first receiving ultrasonic crystal oscillator are arranged along the direction of blood flow velocity, the first receiving ultrasonic crystal oscillator is used to receive a first corresponding ultrasonic signal reflected by a blood vessel from a first ultrasonic signal emitted by the first transmitting ultrasonic crystal oscillator, and the first transmitting ultrasonic crystal oscillator and the first receiving ultrasonic crystal oscillator are arranged at an angle;
The scanning and positioning probe includes a second transmitting ultrasonic crystal oscillator and a second receiving ultrasonic crystal oscillator. The second transmitting ultrasonic crystal oscillator and the second receiving ultrasonic crystal oscillator are arranged in a direction perpendicular to the direction of blood flow rate. The second receiving ultrasonic crystal oscillator is used to receive a second corresponding ultrasonic signal emitted by the second transmitting ultrasonic crystal oscillator and reflected by the blood vessel. The second transmitting ultrasonic crystal oscillator and the second receiving ultrasonic crystal oscillator are arranged at an angle.

As a preferred embodiment of the present application, four second grooves are provided in the second housing, and the four second grooves are used to install a transmitting ultrasonic crystal oscillator, a first receiving ultrasonic crystal oscillator, a second transmitting ultrasonic crystal oscillator, and a second receiving ultrasonic crystal oscillator;
Each of the second grooves is disposed on an isolation boss, and the isolation boss is disposed in the second housing.

As a preferred embodiment of the present application, the monitoring mechanism further includes a fit monitoring mechanism;
The fit monitoring mechanism is arranged on the second shell near the surface of the living body limb and is used to monitor whether the second shell fits the surface of the living body limb.

As a preferred embodiment of the present application, a protrusion is provided on a surface of the second shell close to the living limb, and the protrusion is provided at a position where the first corresponding ultrasonic signal and the second corresponding ultrasonic signal intersect with the surface of the second shell close to the living limb.

As a preferred embodiment of the present application, a sliding member is provided between the supporting mechanism and the second shell.

Compared with the prior art, a blood flow monitoring device provided in an embodiment of the present application is configured to reciprocate a monitoring device by setting a monitoring device on a support mechanism, and the support mechanism is set on the surface of a living limb; the monitoring unit includes a second shell, a flow velocity measurement component and a scanning positioning component, the flow velocity measurement probe and the scanning positioning probe are both set in the second shell, and the second shell is connected to the first shell; the flow velocity measurement probe is used to emit a first ultrasonic signal at a preset inclination angle to the direction of the blood flow velocity and a first corresponding ultrasonic signal reflected after receiving the monitored blood, the blood flow velocity in the blood vessel is measured by the first corresponding ultrasonic signal, and the blood flow rate is calculated by the blood flow velocity and the blood vessel diameter; the scanning positioning probe is used to emit a second ultrasonic signal perpendicular to the direction of the blood flow velocity and the first ultrasonic signal and a second corresponding ultrasonic signal reflected after receiving the monitored blood, and the position and diameter of the blood vessel are monitored and measured by the second ultrasonic signal; the first shell is driven to reciprocate on the support mechanism by the driving mechanism to determine the position of the target blood vessel and to monitor the blood flow in the target blood vessel. Through the flow monitoring device provided in the embodiment of the present invention, the target blood vessel can be accurately found to monitor the blood flow in the blood vessel, and further, long-term continuous monitoring of the blood flow of human limbs can be achieved, and the technical problem of the existing technology that only relies on medical staff to monitor the target blood vessel flow for a long time and brings about a high labor intensity.

### BRIEF DESCRIPTION OF THE DRAWINGS

The drawings described herein are used to provide a further understanding of the present application and constitute a part of the present application. The illustrative embodiments of the present application and their descriptions are used to explain the present application and do not constitute an improper limitation on the present application. Some specific embodiments of the present application will be described in detail in an illustrative and non-restrictive manner with reference to the drawings. The same reference numerals in the drawings indicate the same or similar components or parts. It should be understood by those skilled in the art that these drawings are not necessarily drawn to scale. In the drawings:
FIG. 1 is a schematic diagram of a three-dimensional structure of a blood flow monitoring device provided in an embodiment of the present application, which is arranged on a fixing mechanism;
FIG. 2 is a schematic diagram of the three-dimensional structure of a support mechanism provided in an embodiment of the present application;
FIG. 3 is a front view of a blood flow monitoring device provided in an embodiment of the present application;
FIG. 4 is a cross-sectional view taken along line A-A of FIG. 3;
FIG. 5 is a cross-sectional view taken along line B-B of FIG. 3;
FIG. 6 is a front view of a monitoring unit according to another embodiment;
FIG. 7 is a cross-sectional view of the monitoring unit taken along the line D-D of FIG. 6 ;
FIG. 8 is a cross-sectional view of the monitoring unit of FIG. 7 taken along line F-F of FIG. 7;
FIG. 9 is a diagram showing the principle of blood vessel position and diameter detection calculation:
FIG. 10 is a schematic diagram of the front structure of the second housing;
FIG. 11 is a schematic diagram of the back structure of the second housing;
FIG. 12 is a schematic diagram of the three-dimensional structure of a driving mechanism provided in an embodiment of the present application;
FIG. 13 is a cross-sectional view taken along line C-C of FIG. 3;
FIGS. 14 and 15 are partial schematic diagrams of the monitoring device moving to one extreme position;
FIGS. 16 and 17 are partial schematic diagrams of the monitoring device moving to the other extreme position;

### DETAILED DESCRIPTION

In order to enable those skilled in the art to better understand the present application, the technical solutions in the embodiments of the present application will be clearly and completely described below in conjunction with the drawings in the embodiments of the present application. Obviously, the described embodiments are only embodiments of a part of the present application, not all embodiments. Based on the embodiments in the present application, all other embodiments obtained by ordinary technicians in the field without creative work should fall within the scope of protection of the present application.

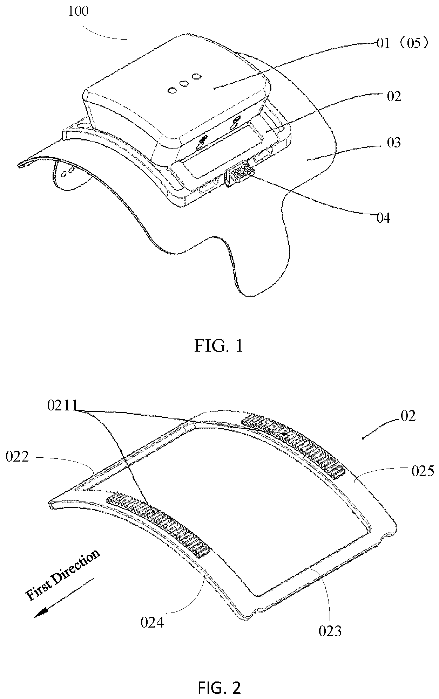

As shown in FIG. 1, the embodiment of the present application provides a blood flow monitoring device 100, including a monitoring equipment 01 and a supporting mechanism 02.

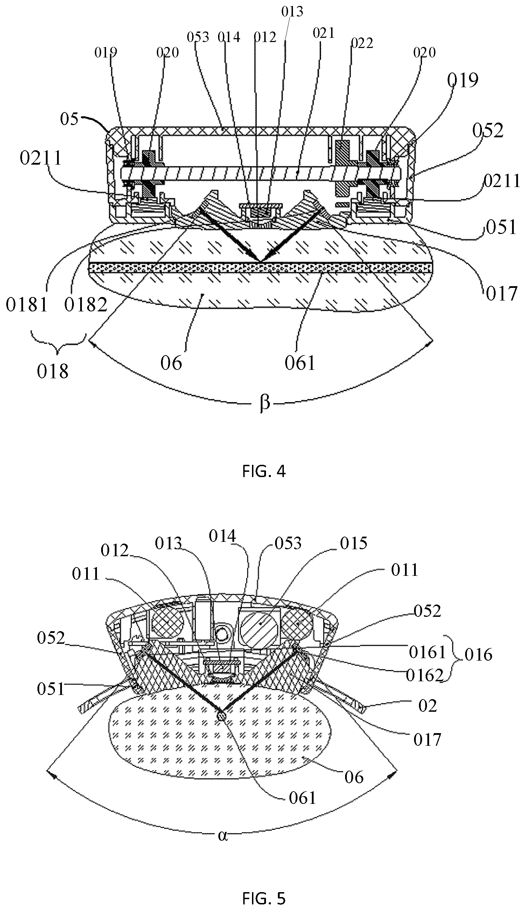

The monitoring equipment 01 reciprocates on the supporting mechanism 02, so that the monitoring equipment 01 can be periodically moved to measure the blood flow, and the monitoring equipment 01 is prevented from being in long-term contact with the surface of the limb 06 (shown in FIG. 5). The supporting mechanism 02 is fixed on the fixing mechanism 03 by the buckle 04, and the fixing mechanism 03 is fixed on the surface of the living limb 06, so that the monitoring equipment 01 can be in close contact with the surface of the limb 06. The blood flow monitoring device 100 of the present application is detachably fixed with the fixing mechanism 03, and is used to monitor the blood flow at the limb 06 of the patient. The limb 06 monitored by the blood flow monitoring device 100 can be the arm, calf, or even the distal part of the limb such as the back of the hand and the back of the foot. The fixing mechanism 03 in the present application can adopt a disposable fixing patch, which makes the monitoring equipment 01 reusable, and the low-cost fixing mechanism 03 is used as a disposable consumable to avoid cross-contact between patients to cause infection. The blood flow monitoring device 100 and the disposable fixing patch 03 in the application are detachably connected.

In addition, the flow rate measurement probe 018 (shown in FIG. 4) in the monitoring equipment 01 of the present application moves automatically along the supporting mechanism 02. When the flow rate needs to be monitored, the flow rate measurement probe 018 can be moved to the surface of the living limb 06 for monitoring. When monitoring is not required, the flow rate measurement probe 018 can be moved away from the surface of the living limb 06. In this way, the flow rate measurement probe 018 can be moved alternately to avoid long-term contact between the flow rate measurement probe 018 and a fixed position of the patient's skin, thereby effectively preventing the surface of the living limb 06 from causing diseases due to airtightness.

FIG. 2, the support mechanism 02 includes a first support member 024, a second support member 022, a third support member 025, and a fourth support member 023 which are sequentially connected to each other, and the first support member 024 is connected to the fourth support member 023. The first support member 024 and the third support member 025 are both arc-shaped and are arranged in a direction perpendicular to the blood flow direction. The first support member 024 and the third support member 025 are both provided with a second transmission mechanism 0211. The second support member 022 and the fourth support member 023 are both arranged in a direction parallel to the blood flow direction, that is, the first direction shown in FIG. 2.

In the embodiment of the present application, the support mechanism 02 is mainly used to support and guide the entire monitoring equipment 01. The support mechanism 02 is a frame-shaped structure as a whole. The support mechanism 02 is detachably connected to the fixing mechanism 03 that is attached to the surface of the limb 06.

The second transmission mechanism 0211 is a driving outer tooth, which can make the entire monitoring equipment 01 move along an arc, so that the monitoring equipment 01 can better fit the limb surface and can avoid air from entering between the monitoring equipment 01 and the limb surface during the movement, thereby affecting the ultrasonic signal measurement. The support mechanism 02 can be made of aluminum alloy or titanium alloy with light density and high strength.

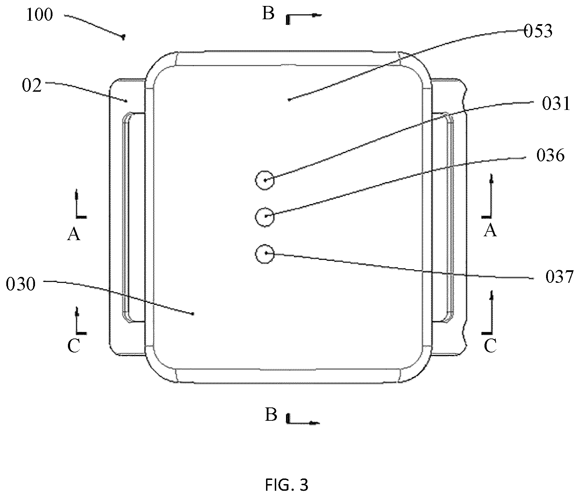

As shown in Figures 1, 3, 4, 5, 12, 13, 14, and 16, the embodiment of the present application utilizes the ultrasonic principle to measure blood flow rate. The monitoring equipment 01 includes a first housing 05 and a monitoring unit 030, a driving mechanism, and a control unit 029 arranged in the first housing 05. The control unit 029 is connected to the monitoring unit 030 and the driving mechanism, respectively. The first housing 05 is movably mounted on the supporting mechanism 02; the control unit 029 is used to send control information to the monitoring unit 030 and the driving mechanism to realize blood flow monitoring. The control unit 029 can also be connected to an external terminal device so as to send the blood flow result monitored by the monitoring device to the external terminal device.

In addition, a battery 011 is also provided in the entire monitoring equipment 01 for supplying power to the control unit 029 and the entire monitoring unit 030.

In addition, the entire monitoring equipment 01 is also provided with a wireless communication module 026, an indicator light, and a charging interface (not shown). The wireless communication module 026 is arranged on the control unit 029, and is used to send the blood flow result monitored by the monitoring equipment 01 obtained by the control unit 029 to an external terminal device, and the external terminal device may be an ECMO host system or a nurse station of a hospital, etc.; the indicator lights include a flow indicator light 031, a battery indicator light 036, and a communication indicator light 037. The flow indicator light 031 is used to indicate normal and abnormal flow conditions and may be indicated in different colors and flashing modes; the battery indicator light 036 is used to indicate the battery charging and discharging status; and the communication indicator light 037 is used to indicate the communication connection status.

The charging interface (not shown) is a positive and negative metal electrode, which can be connected to an external power source to charge the battery 011.

The monitoring unit 030 includes a second shell 017, a flow velocity measurement probe 018, and a scanning positioning probe 016. The flow velocity measurement probe 018 and the scanning positioning probe 016 are both arranged in the second shell 017, and the second shell 017 is connected to the first housing 05. The second shell 017 can slide along the supporting mechanism 02 and perform reciprocating swing scanning, so as to detect the position of the blood vessel 061 and stay at the optimal position for monitoring.

In the embodiment of the present application, the driving mechanism cooperates with the second transmission mechanism 0211 on the supporting mechanism 02 to drive the monitoring unit 030 to move along the supporting mechanism 02.

The flow velocity measurement probe 018 is used to emit a first ultrasonic signal with a preset inclination angle to the direction of the blood flow velocity and receive a first corresponding ultrasonic signal reflected after monitoring the blood, measure the flow velocity of the blood 0062 in the blood vessel 061 through the first corresponding ultrasonic signal, and calculate the blood flow rate through the blood flow velocity and the diameter of the blood vessel 061.

The scanning positioning probe 016 is used to transmit a second ultrasonic signal perpendicular to the direction of blood flow velocity and the first ultrasonic signal and receive a second corresponding ultrasonic signal reflected after monitoring the blood 062, and monitor the position and diameter of the blood vessel 061 through the second ultrasonic signal.

The driving mechanism is connected to the first housing 05, and is used to drive the first housing 05 to reciprocate on the supporting mechanism 02 to determine the position of the target blood vessel 061 and to monitor the flow of blood 062 in the target blood vessel 061.

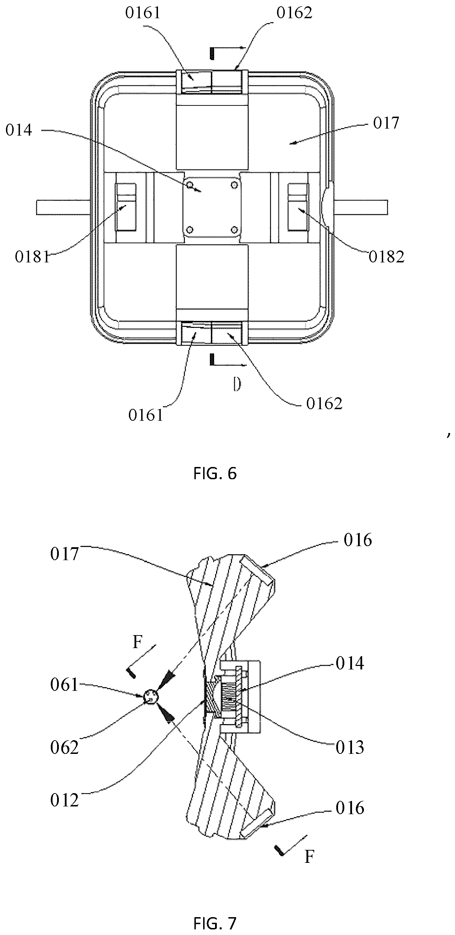

Further, as shown in FIGS. 4 and 6 , the flow velocity measurement probe 018 includes a first transmitting ultrasonic crystal oscillator 0181 and a first receiving ultrasonic crystal oscillator 0182, the first transmitting ultrasonic crystal oscillator 0181 and the first receiving ultrasonic crystal oscillator 0182 are arranged along the direction of blood flow velocity. The first receiving ultrasonic crystal oscillator 0182 is used to receive a first corresponding ultrasonic signal emitted by the first transmitting ultrasonic crystal oscillator 0181 and reflected by the blood vessel 061, and the first transmitting ultrasonic crystal oscillator 0181 and the first receiving ultrasonic crystal oscillator 0182 are arranged at an angle.

That is, the first transmitting ultrasonic crystal oscillator 0181 and the first receiving ultrasonic crystal oscillator 0182 are arranged in parallel along the blood flow direction, and the flow velocity measurement probe 018 is used to measure the blood flow velocity. An ultrasonic signal is emitted by the first transmitting ultrasonic crystal oscillator 0181, which is reflected by the blood in the blood vessel 061 and then received by the other first receiving ultrasonic crystal oscillator 0182. The blood flow velocity can be calculated by the time difference of the received signal using the Doppler effect, and then the blood flow rate is calculated by multiplying the velocity by the cross sectional area of the blood vessel 061 calculated using the diameter measured by the scanning positioning probe 016.

The angle β between the first transmitting ultrasonic crystal oscillator 0181 and the first receiving ultrasonic crystal oscillator 0182 is 50° -110°, or about 82 °.

In FIG. 5, the scanning and positioning probe 016 includes a second transmitting ultrasonic crystal oscillator 0161 and a second receiving ultrasonic crystal oscillator 0162. The second transmitting ultrasonic crystal oscillator 0161 and the second receiving ultrasonic crystal oscillator 0162 are arranged in a direction perpendicular to the direction of blood flow. The second receiving ultrasonic crystal oscillator 0162 is used to receive the second corresponding ultrasonic signal emitted by the second transmitting ultrasonic crystal oscillator 0161 and reflected by the blood vessel 061. The second transmitting ultrasonic crystal oscillator 0161 and the second receiving ultrasonic crystal oscillator 0162 are arranged at an angle α. The angle α between the second transmitting ultrasonic crystal oscillator 0161 and the second receiving ultrasonic crystal oscillator 0162 is 50° -110°, or about 80°.

It should be noted that the second transmitting ultrasonic crystal oscillator 0161 and the second receiving ultrasonic crystal oscillator 0162 are respectively arranged perpendicular to the blood flow direction and are used to scan the blood vessel position and measure the blood vessel diameter. One of the second transmitting ultrasonic crystal oscillators 0161 transmits an ultrasonic signal, which is reflected by the blood in the blood vessel and received by the other second receiving ultrasonic crystal oscillator 0162. The propagation distance can be calculated by the time difference of the received signal, and then the blood vessel position and diameter can be obtained by measuring at different positions and calculating the resulting multiple sets of data. After calculating the blood vessel diameter and position, it is also beneficial to determine whether the monitored blood vessel diameter is comparable to the diameter of the same blood vessel in an average person. If it does not match, it means that the monitored blood vessel position is wrong, and the scanning and monitoring can be re-scanned or an alarm can be issued to notify the user.

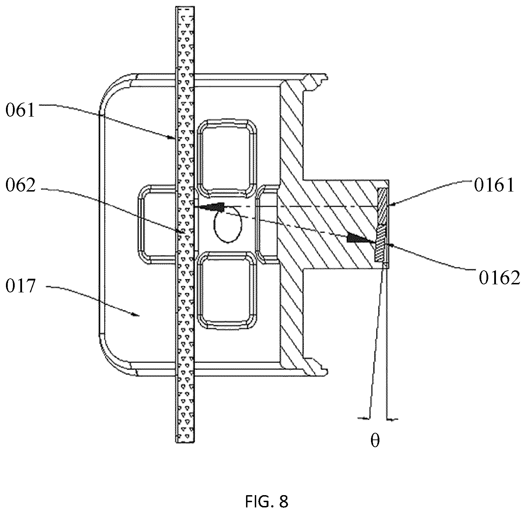

In another embodiment of the present application, as shown in FIGS. 6, 7, and 8, the scanning and positioning probes 016 are divided into two groups and are symmetrically arranged along the direction of blood flow. As such, there are two second transmitting ultrasonic crystal oscillators 0161 and two second receiving ultrasonic crystal oscillators 0162, wherein each group of the scanning and positioning probes 016 includes a second transmitting ultrasonic crystal oscillator 0161 and a second receiving ultrasonic crystal oscillator 0162. The second transmitting ultrasonic crystal oscillator 0161 of the first group and the second receiving ultrasonic crystal oscillator 0162 of the second group are arranged side by side along the direction of blood flow, and the two groups are inclined at a certain angle. The ultrasonic signal emitted by a second transmitting ultrasonic crystal oscillator 0161 in a group can be received by the second receiving ultrasonic crystal oscillator 0162 in that group after being reflected by the blood vessel.

In the embodiment of the present application, the angle α (shown in Figure 5) between the two oscillators 0161, 0162 in each group of the scanning positioning probes 016 is 50°-110°, and the angle θ between the second transmitting ultrasonic crystal oscillator 0161 in one group and the second receiving ultrasonic crystal oscillator 0162 in the other group is 5° - 10°.

In any two scanning and positioning probes 016, one of the second transmitting ultrasonic crystal oscillators 0161 sends out an ultrasonic signal, which is reflected by the blood in the blood vessel and then received by the second receiving ultrasonic crystal oscillator 0162. The propagation distance can be calculated by the time difference of the received signals.

Two groups of relatively fixed scanning and positioning probes 016 can perform measurements at the same position, measure two points of the inner wall of the blood vessel 061 from two different angles, and then obtain the position and diameter of the blood vessel 061 by measuring at different positions and calculating the resulting multiple sets of data. Two sets of relatively fixed scanning and positioning probes 016 can more accurately measure the position of the blood vessel 061, avoiding measurement errors introduced by mobile position deviations. The measurement position of the flow velocity measurement probe 018 can be accurately located by judging whether the distances measured by the two groups of scanning and positioning probes 016 are the same, because when the two distances are equal, the blood vessel 061 will be in the middle of the flow velocity measurement probe 018, so that the flow velocity measurement probe 018 detects the middle position of the blood vessel 061, and the monitored flow velocity is more accurate.

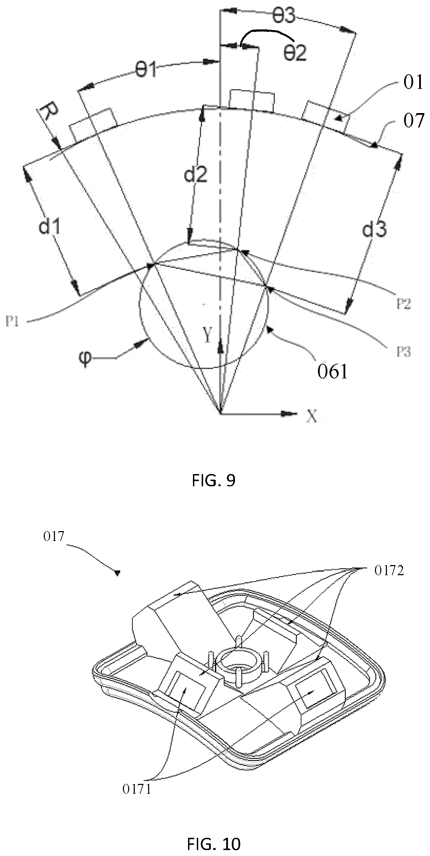

As shown in FIG. 9, the position and diameter detection calculation principle of blood vessel 061 is as follows:

A calculation coordinate system is established, with the center of the movement trajectory 07 of the blood flow monitoring device 100 as the coordinate origin, the middle position as the vertical direction Y axis, and the horizontal direction as the X axis. Since the structural design movement trajectory 07 is an arc, its radius is defined as R. The blood flow monitoring device 100 moves the monitoring equipment 01 to any three offset angles θ1, θ2, and θ3 to monitor the distances d1, d2, and d3 from the inner wall of the blood vessel 061. From the illustrated geometric relationship diagram, the coordinates of the three P1, P2, and P3 measured on the diameter circle of the blood vessel 061 can be calculated as (-(R-d1)sin(θ1), (R-d1)cos(θ1)), ((R-d2)sin(θ2), (R-d2)cos(θ2)), ((R-d3)sin(θ3), and (R-d3)cos(θ3)). The coordinates of the center of the circle can be calculated from the coordinates of the three points on the circle, and the blood vessel diameter can be calculated using the coordinate values of any point P1, P2, P3 and the center of the circle. Assuming that the coordinates of P1 are (X1, Y1) and the coordinates of the center of the circle are (X0, Y0), the diameter can be calculated using the following formula: diameter ϕ = 2*sqrt((X1-X0)² +(Y1-Y0)²).

As shown in FIGS. 4 and 5, the monitoring mechanism also includes a fit monitoring mechanism. The fit monitoring mechanism is disposed on the second shell 017 close to the surface of the living limb 06, and is used to monitor whether the second shell 017 fits the surface of the living limb 06.

A fit monitoring mechanism is provided in the middle of the side of the second shell 017 facing away from the installation groove. In the embodiment of the present application, the fit monitoring mechanism is composed of an infrared monitoring sensor 013 and an infrared monitoring sensor protective cover 012. The infrared monitoring sensor 013 is fixed to the second shell 017 through an infrared monitoring sensor fixing plate 014. The infrared monitoring sensor 013 is mainly used to monitor whether the monitoring unit 030 in the present application fits the skin of the patient being tested. If it is detected to be fitting, the blood vessel 061 monitoring unit 030 will be automatically started.

The second shell 017 is fixed at the lower part of the first housing 05 and can slide along the support mechanism 02 to perform reciprocating swing scanning, so as to detect the position of the blood vessel 061 and stay at the optimal position for monitoring.

Further, as shown in FIG. 10, four second grooves 0171 are provided in the second shell 017, and the four second grooves 0171 are used to install the first transmitting ultrasonic crystal oscillator 0181, the first receiving ultrasonic crystal oscillator 0182, the second transmitting ultrasonic crystal oscillator 0161 and the second receiving ultrasonic crystal oscillator 0162; each of the second grooves 0171 is arranged on the isolation mounting boss 0172, and the isolation mounting boss 0172 is arranged in the second shell 017.

That is to say, the second shell 017 is provided with a plurality of second grooves 0171 for installing and positioning the first transmitting ultrasonic crystal oscillator 0181, the first receiving ultrasonic crystal oscillator 0182, the second transmitting ultrasonic crystal oscillator 0161, and the second receiving ultrasonic crystal oscillator 0162. The isolation mounting bosses 0172 outside the mounting grooves are spaced apart from each other, so as to avoid interference caused by conduction when the first transmitting ultrasonic crystal oscillator 0181 and the second transmitting ultrasonic crystal oscillator 0161 are reflected at the same time.

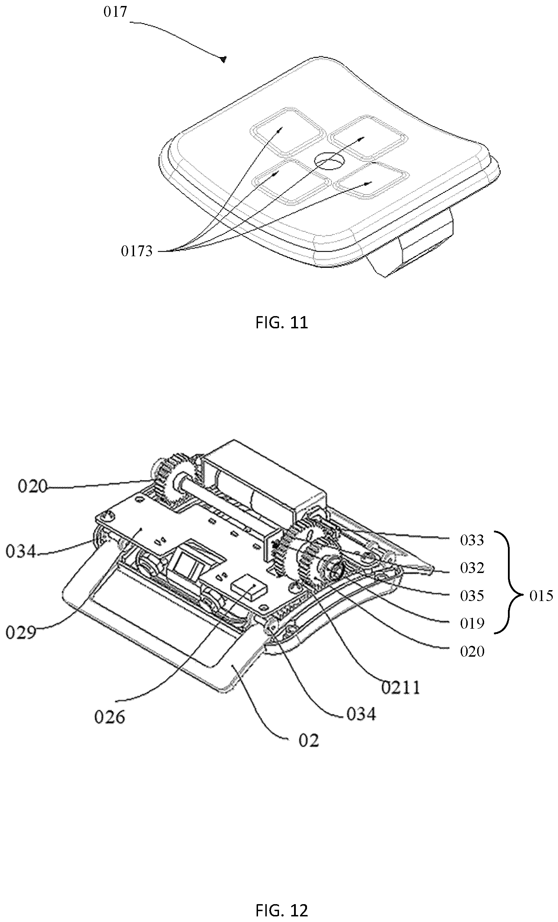

As shown in FIG. 11, a protrusion 0173 is provided on one surface of the second shell 017 close to the living body limb 06, and the protrusion 0173 is provided at a position where the first corresponding ultrasonic signal and the second corresponding ultrasonic signal intersect with the surface of the second shell 017 close to the living body limb 06.

The protrusion 0173 on one side of the surface close to the living limb 06 facilitates the second shell 017 to be in close contact with the skin of the measured part to avoid the generation of bubbles. Once there is a bubble between the two, the ultrasonic signal will immediately attenuate and become very weak after passing through the bubble, making it impossible to monitor.

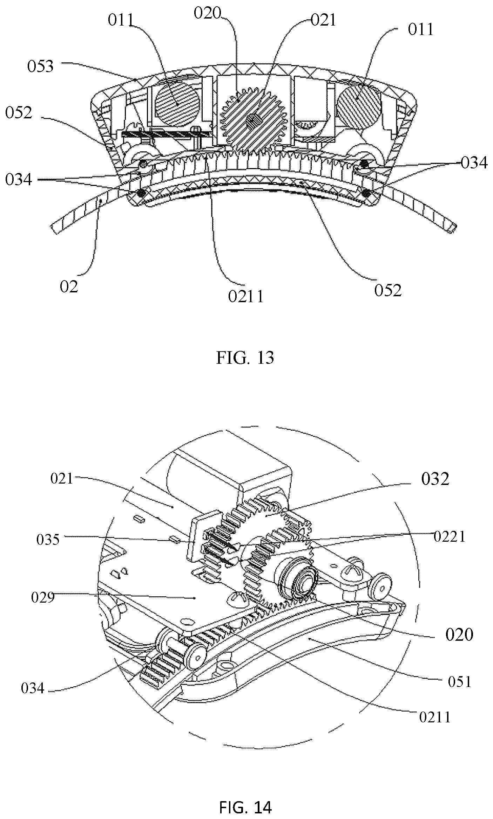

In FIGS. 12 and 13, the driving unit includes a power output mechanism 015 and a first transmission mechanism connected to each other, a second transmission mechanism 0211 is provided on the support mechanism 02, and the monitoring unit 030 and the power output mechanism 015 are electrically connected to the control mechanism respectively. The first transmission mechanism is connected to the first housing 05, and the first transmission mechanism and the second transmission mechanism 0211 are meshedly arranged in that the teeth of the gears in the first transmission mechanism and the second transmission mechanism 0211 are meshed together.

The control unit 029 controls the power output mechanism 015 to drive the first transmission mechanism to move, and the first transmission mechanism cooperates with the second transmission mechanism 0211 to drive the first housing 05 to reciprocate on the support mechanism 02.

Further, the first transmission mechanism includes a first gear 033, a second gear 032, a third gear 020 and a transmission shaft 021. The first gear 033 is connected to the output end of the power output mechanism 015, thereby driving the second gear 032 to rotate through gear transmission, and then the third gear 020 is engaged with the second transmission mechanism 0211, driving the entire monitoring equipment 01 to move along the supporting mechanism 02.

The second gear 032 and the third gear 020 are respectively arranged on the transmission shaft 021 and are both coaxially arranged with the transmission shaft 021. The first gear 033 and the second gear 032 are meshed and arranged. The transmission shaft 021 and the second gear 032 are coaxially arranged.

Both ends of the transmission shaft 021 are rotatably disposed on the inner wall of the first housing 05, and the third gear 020 cooperates with the second transmission mechanism 0211 to drive the monitoring equipment 01 to reciprocate on the supporting mechanism 02.

The two ends of the transmission shaft 021 are fixed to the first housing 05 through bearings 019. The bearings 019 can reduce the transmission resistance and improve the transmission efficiency. The second gear 032 is engaged with the first gear 033 to reduce the speed and improve the output driving torque. The power output mechanism 015 is usually equipped with a reducer to further reduce the output speed and improve the output driving torque. In the embodiment of the present application, the power output mechanism 015 is a motor.

In FIGS. 14-17, a first group of first grooves 0221 and a second group of first grooves 0222 are provided on the second gear 032, and extreme position detection mechanisms are provided at corresponding positions of the two groups of first grooves. The extreme position detection mechanisms are used to detect whether the monitoring equipment 01 moves to the two extreme positions of the supporting mechanism 02.

It should be noted that the first grooves 0221, 0222 are used for extreme position identification, the second group of first grooves 0222 has only one groove, and the first group of first grooves 0221 has two grooves. The two groups of first grooves 0221, 0222 are at different arc positions of the second gear 032, and two photoelectric detection switches 035 are provided at the corresponding positions of the two groups of first grooves 0221, 0222.

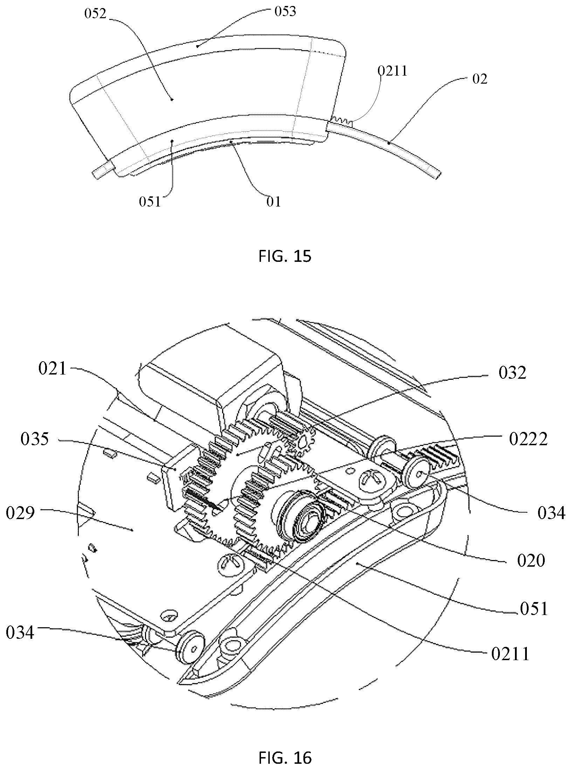

In FIGS. 14 and 15, when the second gear 032 rotates to the position where the first group of first grooves 0221 correspond to the two photoelectric detection switches 035, the two photoelectric detection switches 035 respectively detect two high-level signals. When the second gear 032 rotates to the position where the second group of first grooves 0222 corresponds to one of the photoelectric detection switches 035, the two photoelectric detection switches 035 respectively detect one high-level signal and one low-level signal.

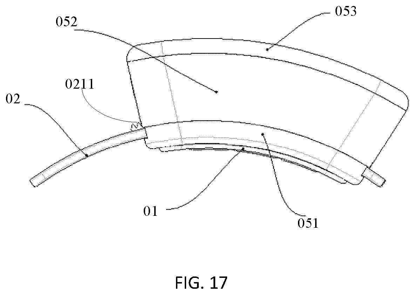

In FIGS. 16 and 17, when the second gear 032 rotates to a position where there is no first groove corresponding to the photoelectric detection switch 035, the two photoelectric detection switches 035 detect two low-level signals respectively. In this way, the photoelectric detection switches 035 detect that the second gear 032 rotates between two groups of first grooves 0221, 0222 positions, and the positions of these two groups of first grooves 0221, 0222 positions are just at the two extreme positions of the device sliding through the transmission ratio calculation design.

The photoelectric detection switch 035 adopts a reflective infrared monitoring sensor 013, on the surface of which is provided with a diode that emits infrared and another diode that receives infrared. When the diode that emits infrared encounters a reflective object, it reflects infrared to the other diode that receives infrared, and it outputs a low-level signal. Otherwise, it outputs a high-level signal. The second gear 032 is made of white plastic or metal material that reflects infrared. Therefore, when it turns to the first groove, infrared is transmitted through the groove, and a high level is output.

As shown in FIG. 14 and FIG. 16, sliding members 034 are provided between the supporting mechanism 02 and the second shell 017.

It should be noted that, as shown in FIG. 4 and FIG. 5, the first housing 05 includes a lower housing 051, a middle housing 052 and an upper housing 053. The lower housing 051 is mainly used to fix the monitoring unit 030 and the infrared monitoring sensor 031. The middle housing 052 is the main body, fixing the main components of the present application such as the drive device, the control unit 029, the battery 011, etc. The upper housing 053 is used to protect the internal components, and a light-transmitting device is provided on the surface, through which the flow indicator light 031, the battery indicator light 036, and the communication indicator light 037 can be seen. A guiding cavity for the support mechanism 02 is provided between the lower housing 051 and the middle housing 052, so that the support mechanism 02 can be installed and connected to the middle housing 052 in a sliding manner. A plurality of sliding members 034 are provided in the cavity, so that the support mechanism 02 does not have sliding friction with the middle housing 052 and the lower housing 051. Instead, there is rolling friction of the sliding member 034, which greatly reduces the amount of friction during the sliding process.

Finally, it should be noted that the above embodiments are only used to illustrate the technical solutions of the present application, rather than to limit it. Although the present application has been described in detail with reference to the aforementioned embodiments, those skilled in the art should understand that they can still modify the technical solutions described in the aforementioned embodiments, or replace some or all of the technical features therein with equivalents. However, these modifications or replacements do not cause the essence of the corresponding technical solutions to deviate from the scope of the technical solutions of the embodiments of the present application.

## Claims

1. A blood flow monitoring device comprises:
a monitoring equipment comprising:
a first housing comprising a first shell;
a monitoring unit comprising:
a second shell connected to the first shell;
a flow velocity measurement probe arranged in the second shell, wherein the flow velocity measurement probe is configured to:
transmit a first ultrasonic signal at a preset inclination angle to a direction of blood flow;
receive a first corresponding ultrasonic signal reflected after monitoring blood;
measure a blood flow velocity in a blood vessel using the first corresponding ultrasonic signal; and
calculate the blood flow rate using the blood flow velocity and a blood vessel diameter; and
a scanning positioning probe arranged in the second shell, wherein the scanning positioning probe is configured to:
transmit a second ultrasonic signal perpendicular to the direction of blood flow;
receive a second corresponding ultrasonic signal reflected after monitoring the blood;
monitor the position and diameter of the blood vessel using the second corresponding ultrasonic signal;
a driving mechanism arranged in the first housing, connected to the first shell, and configured to drive the first shell to reciprocate on the supporting mechanism to determine the position of the blood vessel and to monitor the blood flow in the blood vessel;
a control unit arranged in the first housing, the control unit being connected to the monitoring unit and the driving mechanism, wherein the control unit controls the driving mechanism; and
a supporting mechanism configured to be arranged on a surface of a living body limb; wherein the first housing is movably sleeved on the supporting mechanism; and wherein the first shell reciprocates on the supporting mechanism.

2. The blood flow monitoring device according to claim 1, wherein:
the driving unit comprises a power output mechanism and a first transmission mechanism connected to each other;
a second transmission mechanism is arranged on the supporting mechanism;
the monitoring unit and the power output mechanism are electrically connected to the control mechanism, respectively;
the first transmission mechanism is connected to the first housing;
the first transmission mechanism and the second transmission mechanism include gears that are meshed together;
the control unit controls the power output mechanism to drive the first transmission mechanism to move; and
the first transmission mechanism cooperates with the second transmission mechanism to drive the first housing to reciprocate on the support mechanism.

3. The blood flow monitoring device according to claim 2, wherein the first transmission mechanism comprises a first gear, a second gear, a third gear and a transmission shaft, wherein:
the first gear is connected to an output end of the power output mechanism;
the second gear and the third gear are respectively arranged on the transmission shaft and are both coaxially arranged with the transmission shaft;
the first gear and the second gear are meshed;
both ends of the transmission shaft are rotatably arranged on the inner side wall of the first shell; and
the third gear cooperates with the second transmission mechanism to drive the monitoring equipment to reciprocate on the supporting mechanism.

4. The blood flow monitoring device according to claim 3, wherein:
the second gear includes two groups of first grooves;
extreme position detection mechanisms are arranged at corresponding positions of the two groups of first grooves; and
the extreme position detection mechanisms are used to detect whether the monitoring equipment moves to either one of two extreme positions of the support mechanism.

5. The blood flow monitoring device according to claim 1, wherein:
the supporting mechanism comprises a first supporting member, a second supporting member, a third supporting member and a fourth supporting member which are sequentially connected to each other, and the first supporting member is connected to the fourth supporting member;
the first support member and the third support member are both arc-shaped and are arranged in a direction perpendicular to the direction of blood flow direction;
the first support member and the third support member are each provided with a second transmission mechanism; and
the second supporting member and the fourth supporting member are both arranged in a direction parallel to the direction of blood flow.

6. The blood flow monitoring device according to claim 1, wherein:
the flow velocity measurement probe comprises a group of a first transmitting ultrasonic crystal oscillator and a first receiving ultrasonic crystal oscillator, wherein:
the first transmitting ultrasonic crystal oscillator and the first receiving ultrasonic crystal oscillator are arranged along the direction of blood flow;
the first receiving ultrasonic crystal oscillator is configured to receive a first corresponding ultrasonic signal reflected by the blood vessel from a first ultrasonic signal emitted by the first transmitting ultrasonic crystal oscillator; and
the first transmitting ultrasonic crystal oscillator and the first receiving ultrasonic crystal oscillator are arranged at a first angle; and
the scanning and positioning probe includes a group of a second transmitting ultrasonic crystal oscillator and a second receiving ultrasonic crystal oscillator, wherein:
the second transmitting ultrasonic crystal oscillator and the second receiving ultrasonic crystal oscillator are arranged in a direction perpendicular to the direction of blood flow;
the second receiving ultrasonic crystal oscillator is configured to receive a second corresponding ultrasonic signal emitted by the second transmitting ultrasonic crystal oscillator and reflected by the blood vessel; and
the second transmitting ultrasonic crystal oscillator and the second receiving ultrasonic crystal oscillator are arranged at a second angle.

7. The blood flow monitoring device according to claim 1, wherein:
the scanning and positioning probe comprises two groups of oscillators that are symmetrically arranged along the blood flow direction;
each group of the scanning and positioning probe comprises a second transmitting ultrasonic crystal oscillator and a second receiving ultrasonic crystal oscillator;
the second transmitting ultrasonic crystal oscillator of the first group and the second receiving ultrasonic crystal oscillator of the second group are arranged side by side along the direction of blood flow, and the second transmitting ultrasonic crystal oscillator and the second receiving ultrasonic crystal oscillator;
a first angle between each of the second transmitting ultrasonic crystal oscillator and a second receiving ultrasonic crystal oscillator in each group is between 50°-110°; and
a second angle between the second transmitting ultrasonic crystal oscillator of the first group and the second receiving ultrasonic crystal oscillator of the second group is between 5°-10 °.

8. The blood flow monitoring device according to claim 7, wherein:
the second housing is provided with four second grooves, and installed in the four second grooves are the first transmitting ultrasonic crystal oscillator, the first receiving ultrasonic crystal oscillator, the second transmitting ultrasonic crystal oscillator, and the second receiving ultrasonic crystal oscillator, respectively;
each of the second grooves is disposed on an isolation boss, respectively; and
each of the isolation bosses is disposed in the second housing.

9. The blood flow monitoring device according to claim 7, wherein a protrusion is provided on a surface of the second shell close to the living body limb, and the protrusion is provided at a position where the first corresponding ultrasonic signal and the second corresponding ultrasonic signal intersect with the surface of the second shell close to the living body limb.

10. The blood flow monitoring device according to claim 1, wherein the monitoring mechanism further comprises a fit monitoring mechanism, wherein the fit monitoring mechanism is arranged on the second shell near the surface of the living body limb and is used to monitor whether the second shell fits the surface of the living body limb.
